# EUROPÄISCHE PATENTSCHRIFT

(11) **EP 1 674 066 B1**
(45) Veröffentlichungstag und Bekanntmachung des Hinweises auf die Patenterteilung: **13.08.2008**
(21) Anmeldenummer: 05024094.4
(22) Anmeldetag: 04.11.2005
(51) Int. Cl.: A61K 6/00, A61K 6/083

(54) **Selbstätzende Dentalmaterialien auf Basis von (Meth)acrylamidophosphaten**
Self-etching dental materials based on (meth)acrylamidophosphates
Matériaux dentaires d'auto-gravure à base de (meth)acrylamidophosphates

(30) Priorität: 22.12.2004 DE 102004061925
(43) Veröffentlichungstag der Anmeldung: 28.06.2006
(73) Patentinhaber: Ivoclar Vivadent AG, 9494 Schaan (LI)
(72) Erfinder: Moszner, Norbert, Prof. Dr., 9495 Triesen (LI); Lamparth, Iris, Dr., 9472 Grabs (CH); Zeuner, Frank, Dr., 9488 Schellenberg (LI); Salz, Ulrich, Dr., 88131 Lindau (DE); Mücke, Angela, Dr., 86199 Augsburg (DE); Zimmermann, Jörg, Dr., 8046 Zürich (CH); Angermann, Jörg, Dr., 7320 Sargans (CH); Rheinberger, Volker, Dr., 9490 Vaduz (LI)
(74) Vertreter: UEXKÜLL & STOLBERG

(56) Entgegenhaltungen:
- EP-A- 0 333 503
- EP-A- 0 712 622

## Beschreibung

Die vorliegende Erfindung betrifft (Meth)acrylamidphosphate mit hoher Hydrolysebeständigkeit, die sich besonders als Haftkomponente für selbstätzende Dentalmaterialien wie Adhäsive, Beschichtungsmaterialien und Komposite eignen.

In der restaurativen Zahnheilkunde finden heutzutage zunehmend selbstätzende, selbstkonditionierende Dentin-Schmelz-Adhäsive Anwendung. Diese Adhäsive sind meist so aufgebaut, daß sie ein Haftmonomer mit Säurefunktion, ein oder mehrere nicht saure Comonomere, Lösungsmittel, einen Polymerisationsinitiator und gegebenenfalls weitere Additive enthalten. Dabei kommen als polymerisationsfähige Haftmonomere vor allem solche sauren Monomere in Frage, die einerseits eine hohe Reaktivität bei der radikalischen Polymerisation zeigen und andererseits in der Lage sind, hinreichend schnell die Zahnhartsubstanz zu konditionieren. Bekannte Beispiele für solche sauren Monomeren sind Carbonsäuremethacrylate, wie 4-MET (4-Methacryloyloxyethyloxycarbonyl-phthalsäure), MAC-10 (10-Methacryloyloxydecylmalonsäure) oder saure Methacrylatphosphate, wie MDP (10-Methacryloyloxydecyldihydrogenphosphat) oder MEP (2-Methacryloyloxyethyldihydrogenphosphat):

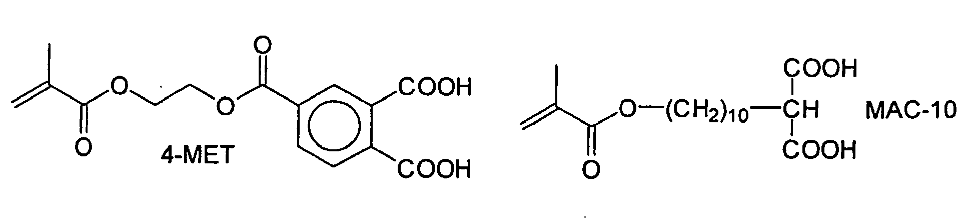

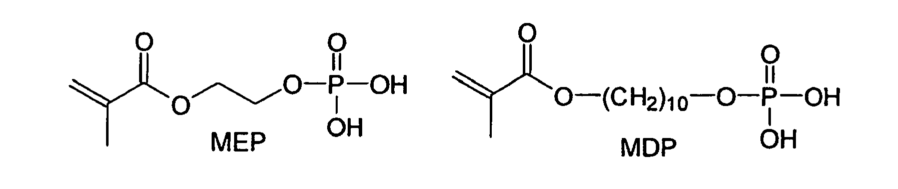

In diesem Zusammenhang sind auch saure Dimethacrylate bekannt, die sich wie PMDM (Additionsprodukt von 1 mol Pyromellithsäureanhydrid mit 2 mol 2-Hydroxyethylmethacrylat) oder GDMP [(1,3-Dimethacryloyloxy)-prop-2-yl-dihydrogenphosphat] aufgrund ihrer vernetzenden Eigenschaften durch eine hohe Polymerisationsaktivität auszeichnen:

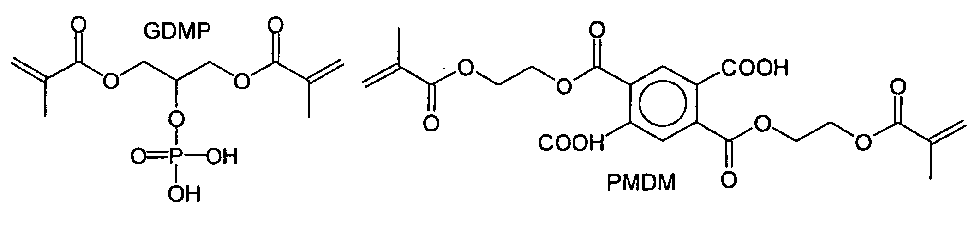

Wasser wird meist als Lösungsmittel oder Cosolvens in Schmelz-Dentin-Adhäsiven eingesetzt, da es die Benetzung der Zahnhartsubstanz fördert. Die Esterbindungen der Methacrylate werden aber bekanntlich unter sauren, wäßrigen Bedingungen hydrolysiert. Durch die Hydrolyse der Monomere verliert das entsprechende Adhäsiv seine Funktion, und die Haftwirkung läßt im Laufe der Zeit deutlich nach. Deshalb werden die sauren Monomere wasserfrei und meist von den anderen Adhäsivbestandteilen separat aufbewahrt. Sie werden entweder erst kurz vor der Anwendung mit dem wäßrigen Teil gemischt oder aber separat auf die Zahnoberfläche appliziert. Es wäre von großem Vorteil, wenn hydrolysestabile Adhäsive hergestellt werden könnten, die alle Komponenten in einer Zusammensetzung vereinigen.

Aus der EP 0 909 761 A1 und der DE 102 34 326 sind hydrolyse-stabile Acrylphosphonsäuren bekannt, in denen polymerisierbare (Meth)acrylgruppen über Ether-, Thioether- oder über Kohlenstoff-Kohlenstoff-Bindungen an den Rest des Moleküls gebunden sind.

Die EP 1 169 996 A1 offenbart Dentalmaterialien auf der Basis von (Meth)acrylamidphosphonsäuren, die aufgrund einer verhältnismäßig langkettigen Brücke zwischen Phosphonsäuregruppe und reaktiver Doppelbindung eine hohe Hydrolysestabilität und verbesserte Hafteigenschaften aufweisen sollen.

Die WO 03/035013 und die WO 03/013444 offenbaren selbstätzende, selbstkonditionierende Dentaladhäsive auf der Basis von Säuregruppen enthaltende (Meth)acrylamiden, wobei Sulfonsäure- und Phosphonsäuregruppen enthaltende Monomere bevorzugt sind. Diese Verbindungen sind synthetisch nur schwer zugänglich.

Aus der EP 0 333 503 A2 sind härtbare Zusammensetzungen bekannt, die Füllstoff enthalten, der mit polymerisierbaren organischen Phosphor- oder Phosphonsäureverbindungen behandelt wurde. Die Zusammensetzungen können zusätzlich acide Monomere enthalten.

Der Erfindung liegt die Aufgabe zugrunde, ein Dentalmaterial bereitzustellen, das sich mittels radikalischer Polymerisation aushärten läßt und dabei eine hohe Polymerisationsneigung zeigt, das in Gegenwart von Wasser bei Raumtemperatur hydrolyseunempfindlich und damit lagerstabil ist und das in der Lage ist, die Zahnhartsubstanz anzuätzen.

Die Aufgabe wird erfindungsgemäß durch polymerisierbare Dentalwerkstoffe gelöst, die mindestens ein (Meth)acrylamidphosphat der folgenden allgemeinen Formel (I) oder ein Pyrophosphat davon enthalten: 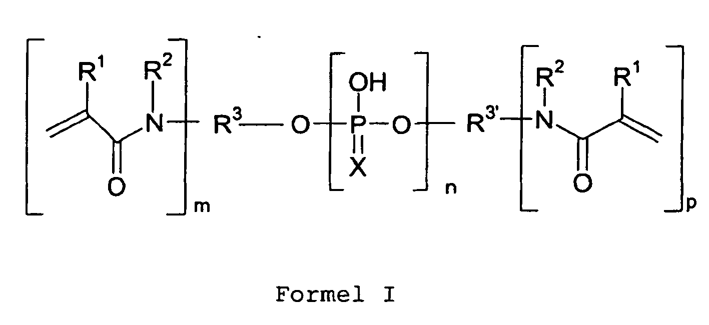 in der
- **R¹**: H oder CH₃ ist;
- **R²**: H oder ein C₁-C₄-Alkylrest ist oder zusammen mit dem Stickstoffatom, an das es gebunden ist, und einem oder mehreren Atomen, die zu R³ bzw. R^{3'} gehören, einen heterocyclischen Ring bildet;
- **R³, R^{3'}**: unabhängig voneinander ein m+n-wertiger bzw. p+n- wertiger, linearer oder verzweigter aliphatischer C₁-C₅₀₋Rest, ein m+n-wertiger bzw. p+n-wertiger aromatischer C₆₋C₁₈-Rest oder ein m+n-wertiger bzw. p+n-wertiger cycloali-phatischer, araliphatischer oder heterocyclischer C₃-C₁₈₋Rest sind, wobei die Kohlenstoffketten des oder der Reste durch O, S, CONR⁴, OCONH unterbrochen sein können, oder zusammen mit einem oder mehreren Atomen, die zu R² gehören und dem Stickstoffatom, an das R² gebunden ist, einen he-terocyclischen Ring bilden, wobei R^{3'} H ist, wenn p = 0, und wobei
- **R⁴**: H, C₁-C₁₀-Alkyl, C₆-C₁₂-Aryl, C₆-C₁₀-Aralkyl oder ein bicyclischer C₄-C₁₂-Rest ist;
- **n**: 1, 2, 3 oder 4 ist, wenn p = 0, und 1 oder 2 ist, wenn p ≠ 0;
- **m**: 1, 2, 3 oder 4 ist;
- **p**: 0, 1, 2, 3 oder 4 ist;
- **X**: O oder S ist.

Vorzugsweise ist R² von H verschieden.

Unter araliphatischen oder Aralkyl-Resten werden Gruppen verstanden, die sowohl aromatische als auch aliphatische Reste enthalten. Ein Typisches Beispiel für einen araliphatischen Rest ist der Benzylenrest -Ph-CH₂- der eine aromatische Phenylengruppe und einen aliphatischen Methylenrest umfaßt.

Der Begriff, Kohlenstoffkette, die durch andere Gruppen und/oder Atome unterbrochen ist, ist so zu verstehen, daß diese anderen Gruppen oder Atome in die Kohlenstoffkette eingeschoben werden, d.h. beidseitig von Kohlenstoffatomen gebunden werden. Diese Gruppen oder Atome können somit keine endständige Position einnehmen. Werden mehrere Gruppen oder Atome in eine Kohlenstoffkette integriert, müssen sie jeweils durch mindestens ein Kohlenstoffatom voneinander getrennt sein. Unter "Kohlenstoffkette" werden keine ringförmigen Molekülgruppen verstanden. Die Gesamtzahl der Gruppen und/oder Atome, die in die Kohlenstoffkette integriert werden, ist mindestens um den Wert 1 kleiner als die Zahl der Kohlenstoffatome in der Kette.

Die Variablen der obigen Formel (I) können unabhängig voneinander folgende bevorzugte Bedeutungen haben:
- **R¹** =: H und/oder CH₃, wobei im Falle m > 1 die R¹-Reste unterschiedliche Bedeutungen haben können;
- **R²** =: H oder ein C₁-C₂₀-Alkylrest oder R² bildet zusammen mit dem Stickstoffatom, an das es gebunden ist, und einem oder mehreren Atomen, die zu R³ bzw. R^{3'} gehören, einen heterocyclischen Ring, vorzugsweise einen Ring mit einem Stickstoffatom und 4 bis 7 Kohlenstoffatomen, insbesondere einen Piperidinylring;
- **R³, R³'** =: unabhängig voneinander ein m+n-wertiger bzw. n+p-wertiger linearer oder verzweigter aliphatischer C₁-C₁₀₋Rest, ein m+n-wertiger bzw. n+p-wertiger aromatischer C₆₋C₁₀-Rest oder ein m+n-wertiger bzw. n+p-wertiger C₅-C₈ cycloaliphatischer oder araliphatischer C₆-C₁₂-Rest, wobei die Kohlenstoffketten des oder der Reste durch O unterbrochen sein können, oder R³ und/oder R^{3'} bilden zusammen mit einem oder mehreren Atomen, die zu R² gehören, und dem Stickstoffatom, an das R² gebunden ist, einen heterocyclischen Ring, vorzugsweise einen Ring mit einem Stickstoffatom und 4 bis 7 Kohlenstoffatomen, insbesondere einen Piperidinylring, wobei R^{3'} H ist, wenn p = 0;
- **R⁴** =: H, C₁-C₆-Alkyl, C₆-Aryl;
- **n** =: 1 oder 2, wenn p = 0, und 1, wenn p ≠ 0;
- **m** =: 1 oder 2;
- **p** =: 0, 1 oder 2;
- **X** =: O.

Ganz besonders bevorzugte Bedeutungen der Variablen der obigen Formel (I) sind:
- **R¹** =: H oder CH₃;
- **R²** =: H oder ein C₁-C₃-Alkylrest oder R² bildet zusammen mit dem Stickstoffatom, an das es gebunden ist, und einem oder mehreren Atomen, die zu R³ gehören, einen Piperidinylring;
- **R³** =: ein m+n-wertiger linearer oder verzweigter aliphatischer C₁-C₃-Rest oder R³ bildet zusammen mit einem oder mehreren Atomen, die zu R² gehören, und dem Stickstoffatom, an das R² gebunden ist, einen Piperidinylring, ganz besonders bevorzugt ein C₃-Alkylenrest;
- **R^{3'}**: = H;
- **R⁴** =: H, C₁-C₃-Alkyl;
- **n** =: 1;
- **m** =: 1 oder 2;
- **p** =: 0;
- **X** =: O.

Verbindungen der Formel (I), bei denen mehrere und insbesondere alle Variablen eine der bevorzugten und insbesondere der besonders bevorzugten Bedeutungen haben, sind erfindungsgemäß besonders geeignet.

Die erfindungsgemässen (Meth)acrylamidphosphate der allgemeinen Formel I (p=0; R^{3'}=H; n=1) lassen sich beispielsweise in 3 Stufen ausgehend von reaktiven (Meth)acrylsäurederivaten (Chlorid, Anhydrid, Ester, Säure) und Aminoalkanolen herstellen. Reaktive (Meth)acrylsäurederivate und Monoaminoalkanole sind kommerziell verfügbar. Polyaminoalkohole (m>1, R²=Alkyl, Aryl etc.) erhält man z.B. durch Umsetzung von Polyhalogenalkoholen mit einem großen Überschuß eines aliphatischen oder aromatischen Amins (B. J. Gaj and D. R. Moore, Tetrahedron Lett. 23 (1967) 2155).

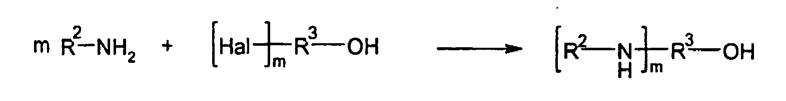

### Konkretes Beispiel:

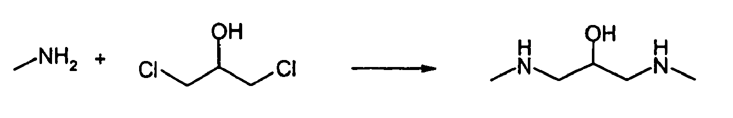

Weiterhin können Diaminoalkohole (m=2, n=1, R² = Alkyl, Aryl etc.) durch Umsetzung von aliphatischen oder aromatischen Aminen mit Epichlorhydrin gewonnen werden (B. J. Gaj and D. R. Moore, Tetrahedron Lett. 23 (1967) 2155).

### Konkretes Beispiel:

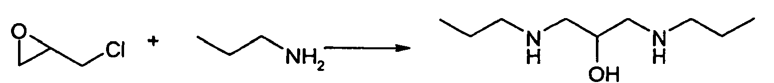

Eine Möglichkeit zur Herstellung von Polyaminopolyolen (m,n > 1) ist die Umsetzung von Polyaminen mit Epoxiden.

### Konkretes Beispiel:

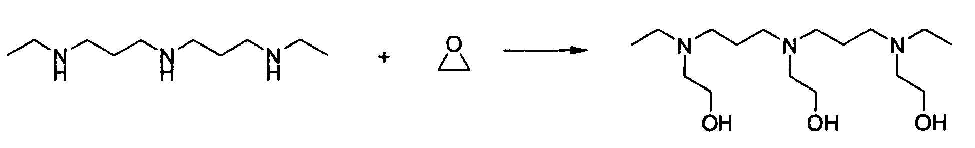

In der 1. Reaktionsstufe wird ein reaktives (Meth) acrylsäurederivat (R¹ = H, CH₃; R' = Hal, O-CO-CR¹=CH₂, OAlkyl, OH) mit Aminoalkanolen unter Anwendung der aus der organischen Chemie bekannten Methoden für die Knüpfung von Amidbindungen (vgl. Methoden der Organischen Chemie, HOUBEN-WEYL Band E5 1985, Georg Thieme Verlag Seiten 941 ff.) zu (Meth)acrylamidoalkoholen umgesetzt.

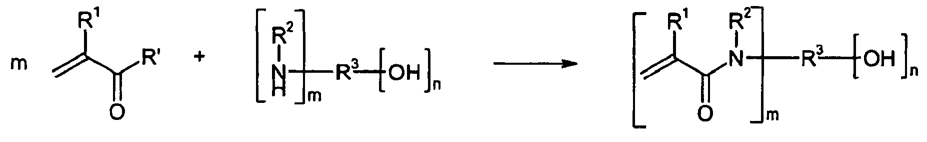

Dazu wird im Falle m = n =1 das reaktive (Meth)acrylsäurederivat mit primären (R² = H) oder sekundären (R² = Alkyl, Aryl) Monoaminoalkanolen, im Falle m > 1, n = 1 mit primären (R² = H) oder sekundären (R²= Alkyl, Aryl) Polyaminoalkanolen, im Falle m = 1, n > 1 mit primären (R² = H) oder sekundären (R²= Alkyl, Aryl) Aminopolyolen und im Falle m > 1, n > 1 mit primären (R² = H) oder sekundären (R²= Alkyl, Aryl) Polyaminopolyolen umgesetzt.

Bevorzugt verwendet man die entsprechenden Säurechloride in Gegenwart äquimolarer Mengen an Base.

### Konkretes Beispiel:

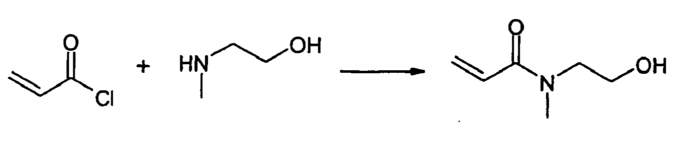

In der 2. Stufe werden die (Meth)acrylamidoalkohole mit n Äquivalenten einer Phosphorylverbindung vorzugsweise Dimethylchlorphosphat (R⁵ = CH₃) umgesetzt (Y.Xu., G. D. Prestwich, J. Org. Chem. 67 (2002) 7158).

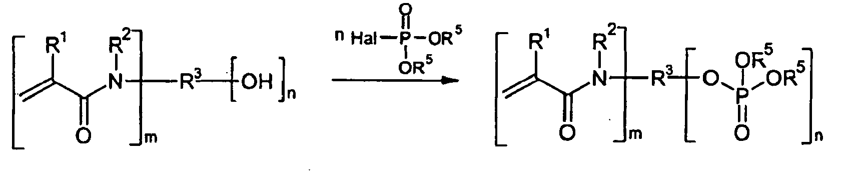

### Konkretes Beispiel:

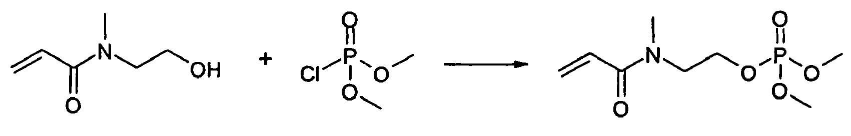

In der 3. Stufe erfolgt die selektive Hydrolyse der Alkoxygruppen OR⁵, vorzugsweise verwendet man ein Dimethylphosphat (R⁵ = CH₃) bei dem die Abspaltung mit Bromtrimethylsilan durchgeführt wird (Y. Xu., G. D. Prestwich, J. Org. Chem. 67 (2002) 7158).

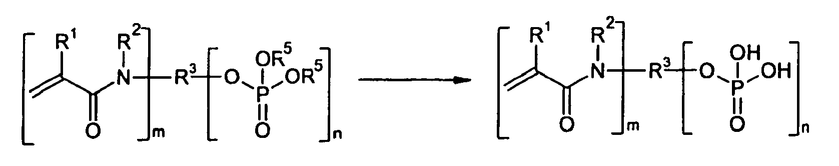

### Konkretes Beispiel:

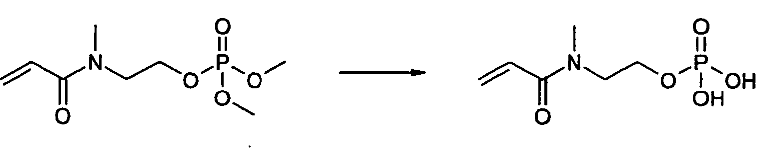

Bevorzugte Beispiele für die erfindungsgemäßen (Meth)acrylamidphosphate der Formel I sind:

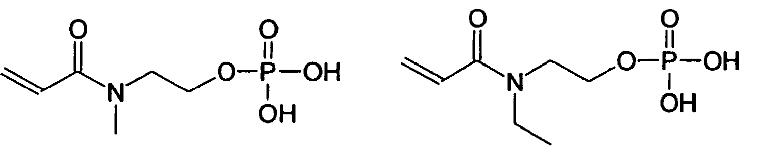

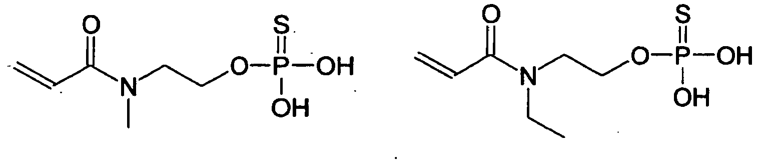

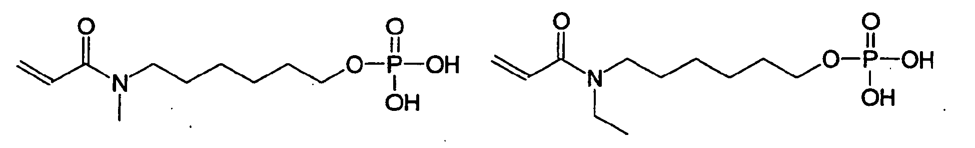

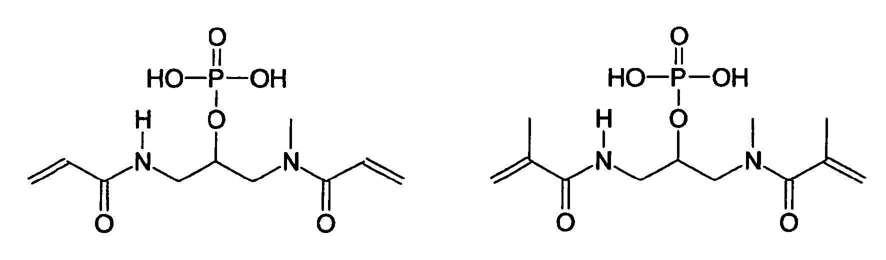

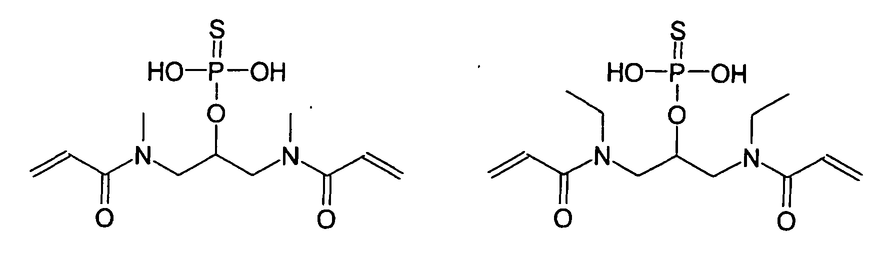

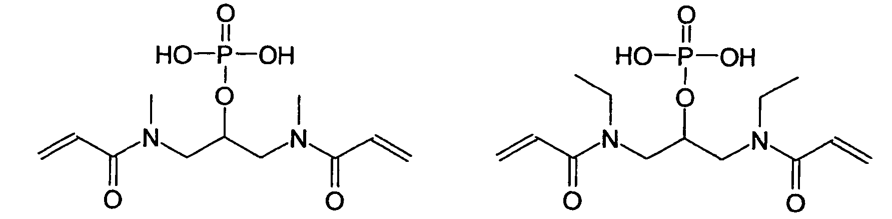

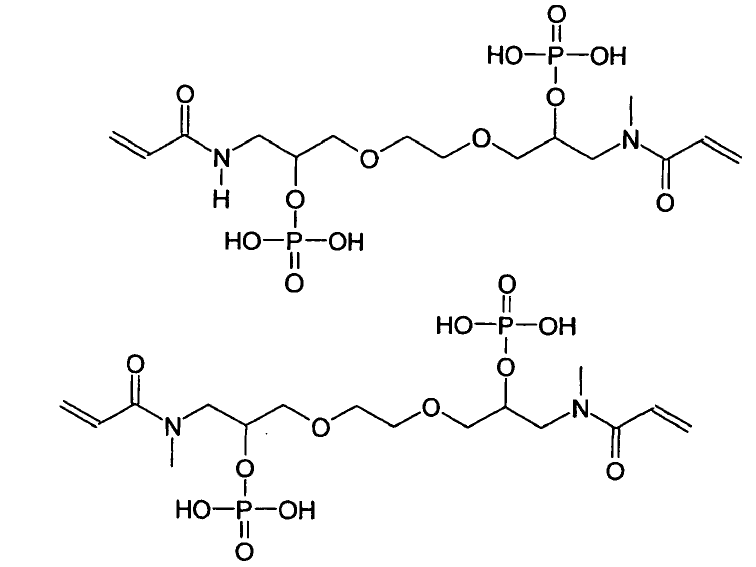

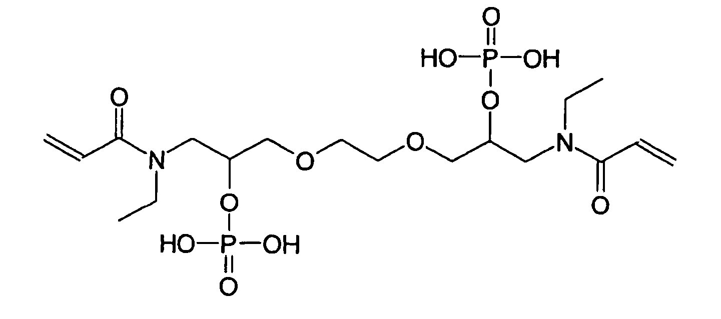

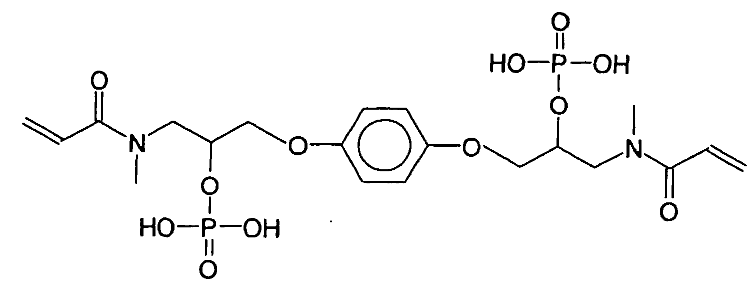

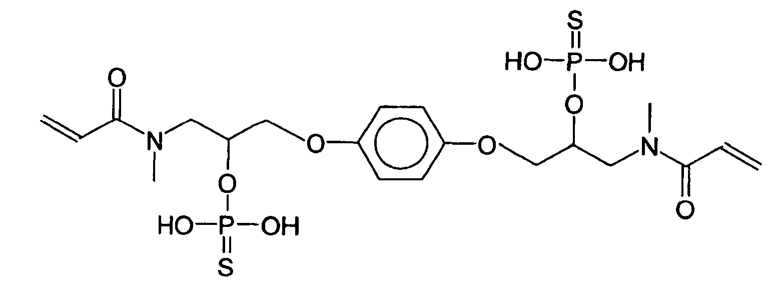

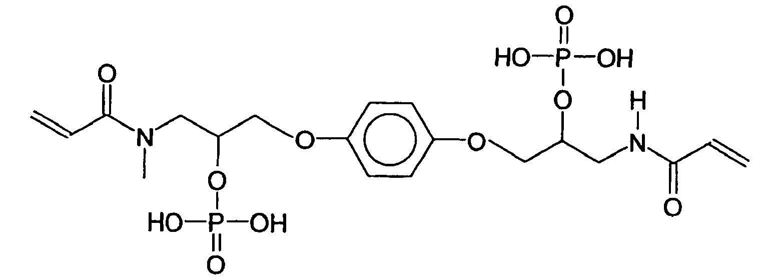

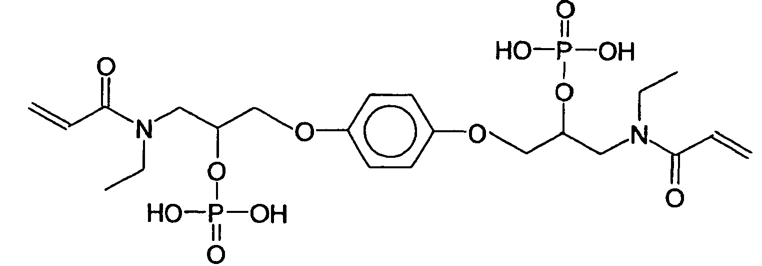

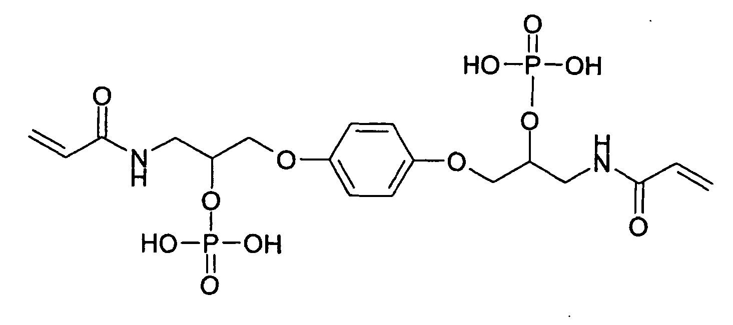

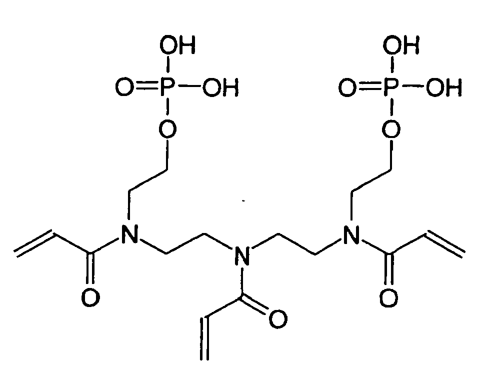

Die erfindungsgemäßen (Meth)acrylamidphosphate der Formel (I) sind stark sauer und sehr gut in Wasser oder Mischungen von Wasser mit polaren Lösungsmitteln, wie Aceton, Ethanol, Acetonitril oder Tetrahydrofuran (THF) löslich. Sie sind in der Lage Zahnschmelz und Dentin vergleichbar mit der Phosphorsäure zu ätzen.

Die (Meth)acrylamidphosphate der Formel (I) sind unter wäßrig sauren Bedingungen bei Raumtemperatur über lange Zeit hydrolysestabil. Im Zusammenhang mit der vorliegenden Erfindung werden solche Verbindungen als hydrolysestabil bezeichnet, die in Wasser oder in Mischungen von Wasser und wassermischbaren Lösungsmitteln bei einer Konzentration von ca. 20 Gew.-% und einem pH-Wert von ca. 2,0 bei 37 °C für mindestens 6 Wochen stabil sind, d.h. zu weniger als 10 %, vorzugsweise weniger als 5 % hydrolysieren.

Aufgrund der hohen Hydrolysestabilität der erfindungsgemäßen (Meth)acrylamidphosphate lassen sich daraus bei Raumtemperatur lagerstabile Mischungen mit Wasser und anderen hydrolysestabilen Komponenten herstellen. Diese Mischungen zeigen in Abhängigkeit von der Struktur und Konzentration der (Meth)acrylamidphosphate der Formel I einen pH-Wert, der zwischen etwa 0,5 und 3,0 liegt und sind deshalb in der Lage die Oberfläche der Zahnhartsubstanz (Schmelz und Dentin) anzuätzen. Diese Mischungen eignen sich daher insbesondere als Adhäsive oder Zemente für den Dentalbereich, ganz besonders als selbstätzende Schmelz-Dentin-Adhäsive. Dabei sind solche (Meth)acrylamidphosphate, die drei (m+p=3) und ganz besonders bevorzugt zwei (polymerisationsfähige (Meth)acrylamidgruppen (m+p=2) enthalten, von besonderem Vorteil.

Die erfindungsgemäßen Dentalwerkstoffe enthalten neben den (Meth)acrylamidphosphaten der Formel (I) vorzugsweise zusätzlich ein Lösungsmittel, besonders bevorzugt Wasser oder eine Mischung von Wasser und einem mit Wasser mischbaren Lösungsmittel. Bevorzugte mit Wasser mischbare Lösungsmittel sind polare Lösungsmitteln, wie Aceton, Ethanol, Acetonitril, Tetrahydrofuran (THF) und Mischungen daraus.

Weiterhin können die erfindungsgemäßen Dentalwerkstoffe auch ein oder mehrere nicht acide, radikalisch polymerisierbare Monomere, wie mono- oder vorzugsweise mehrfach funktionelle (Meth)acrylverbindungen enthalten. Unter monofunktionellen (Meth)acrylverbindungen werden Verbindungen mit einer, unter mehrfach funktionellen (Meth)acrylverbindungen Verbindungen mit zwei oder mehr, vorzugsweise 2 bis 3 (Meth)acrylgruppen verstanden.

Bevorzugte monofunktionelle (Meth)acrylverbindungen sind hydrolysestabile Mono(meth)acrylate, z.B. Mesityl(meth)acrylat, oder 2-(Alkoyxymethyl)acrylsäuren, z.B. 2-(Ethoxymethyl)acrylsäure, 2-(Hydroxymethyl)acrylsäure, N-mono- oder N-disubstituierte Acrylamide, wie z.B. N-Ethylacrylamid, N,N-Dimethylacrylamid, N-(2-Hydroxyethyl)acrylamid oder N-Methyl-N-(2-hydroxyethyl)acrylamid, N-monosubstituierte Methacrylamide, wie z.B. N-Ethylmethacrylamid oder N-(2-Hydroxyethyl)methacrylamid, sowie außerdem N-Vinylpyrrolidon oder Allylether und Mischungen dieser Substanzen. Die oben genannten Monomere sind bei Raumtemperatur flüssig oder niedrigschmelzend, d.h. sie haben einen Schmelzpunkt von weniger als 60 °C, und können daher als Verdünner eingesetzt werden.

Bevorzugte mehrfach funktionelle (Meth)acrylate sind z.B. hydrolysestabile Urethane aus 2-(Hydroxymethyl)acrylsäure und Diisocyanten, wie 2,2,4-Trimethylhexamethylendiisocyanat oder Isophorondiisocyanat, vernetzende Pyrrolidone, wie z.B. 1,6-Bis(3-vinyl-2-pyrrolidonyl)-hexan, oder Bisacrylamide, wie Methylen- oder Ethylenbisacrylamid, oder Bis(meth)acrylamide, wie z.B. N,N'-Diethyl-1,3-bis(acrylamido)-propan, 1,3-Bis(methacrylamido)-propan, 1,4-Bis(acrylamido)-butan oder 1,4-Bis(acryloyl)-piperazin, die durch Umsetzung aus den entsprechenden Diaminen mit (Meth)acrylsäurechlorid synthetisiert werden können. Die übrigen Verbindungen sind meist kommerziell zugänglich. Mehrfach funktionelle (Meth)acrylate wirken aufgrund der Anzahl der polymerisierbaren Gruppen bei der Polymerisation als Vernetzer. Mischungen dieser Substanzen und Mischungen mit monofunktionellen (Meth)acrylaten sind ebenfalls geeignet.

Zur Initiierung der radikalischen Polymerisation enthalten die Dentalwerkstoffe vorzugsweise einen Initiator, besonders bevorzugt einen Photoinitiator.

Als Photoinitiatoren werden vorzugsweise Benzophenon, Benzoin sowie deren Derivate oder α-Diketone oder deren Derivate wie 9,10-Phenanthrenchinon, 1-Phenyl-propan-1,2-dion, Diacetyl oder 4,4-Dichlorbenzil eingesetzt. Besonders bevorzugt werden Campherchinon und 2,2-Methoxy-2-phenyl-acetophenon und insbesondere α-Diketone in Kombination mit Aminen als Reduktionsmittel, wie z.B. 4-(Dimethylamino)-benzoesäureester, N,N-Dimethylaminoethylmethacrylat, N,N-Dimethyl-sym.-xylidin oder Triethanolamin, verwendet.

Als Initiatoren für eine bei Raumtemperatur durchgeführte Polymerisation werden Redox-Initiatorkombinationen, wie z.B. Kombinationen von Benzoylperoxid mit N,N-Dimethylsym.-xylidin oder N,N-Dimethyl-p-toluidin, verwendet. Darüber hinaus sind auch Redoxsysteme bestehend aus Peroxiden und solchen Reduktionsmitteln, wie z.B. Ascorbinsäure, Barbituraten oder Sulfinsäuren, besonders geeignet.

Weiterhin können die erfindungsgemäßen Dentalwerkstoffe zur Verbesserung der mechanischen Eigenschaften oder zur Einstellung der Viskosität mit organischen oder anorganischen Partikeln gefüllt werden. Bevorzugte anorganische partikuläre Füllstoffe sind amorphe kugelförmige Materialien auf der Basis von Oxiden, wie ZrO₂ und TiO₂ sowie Mischoxiden aus SiO₂, ZrO₂ und/oder TiO₂, nanopartikuläre oder mikrofeine Füllstoffe, wie pyrogene Kieselsäure oder Fällungskieselsäure sowie Minifüllstoffe, wie Quarz-, Glaskeramik- oder Glaspulver mit einer durchschnittlichen Teilchengröße von 0,01 bis 1 µm sowie röntgenopake Füllstoffe, wie Ytterbiumtrifluorid oder nanopartikuläres Tantal(V)-oxid bzw. Bariumsulfat.

Dentalwerkstoffe zur Verwendung als Schmelz-Dentin-Adhäsive enthalten als Füllstoffe vorzugsweise nanopartikuläre (Primärpartikeldurchmesser von 1 bis 100 nm) amorphe kugelförmige Materialien auf der Basis von Oxiden, wie ZrO₂ und TiO₂ bzw. Mischoxiden aus SiO₂, pyrogene Kieselsäure oder Fällungskieselsäure, ZrO₂ und/oder TiO₂, sowie röntgenopake nanopartikuläre Füllstoffe, wie Ytterbiumtrifluorid, Tantal(V)-oxid oder Bariumsulfat.

Um die selbstkonditionierende Wirkung der erfindungsgemäß eingesetzten (Meth)acrylamidphosphate der Formel (I) zu erhalten, ist es wesentlich, daß die (Meth)acrylamidphosphate nicht auf den Füllstoff aufgebracht werden, da hierbei die Phosphorsäuregruppen von der Füllstoffoberfläche gebunden werden.

Darüber hinaus können die erfindungsgemäßen Zusammensetzungen weitere Additive enthalten, wie z.B. Stabilisatoren, Aromastoffe, mikrobiocide Wirkstoffe, fluoridionenabgebende Additive, optische Aufheller, Weichmacher oder UV-Absorber.

Die (Meth)acrylamidphosphate der Formel (I) eignen sich besonders zur Herstellung von Dentalwerkstoffen, insbesondere Adhäsiven, Beschichtungsmaterialien und Kompositen für dentale Zwecke.

Die (Meth)acrylamidphosphate der Formel (I) eignen sich ganz besonders zur Herstellung von Dentaladhäsiven und selbsthaftenden Befestigungszementen, insbesondere zur Herstellung von Schmelz-Dentin-Adhäsiven. Solche Adhäsive und Zemente zeichnen sich durch eine sehr gute Haftung auf der Zahnhartsubstanz aus und sind unter feuchten Bedingungen hydrolysestabil.

Erfindungsgemäß sind besonders solche Dentalwerkstoffe bevorzugt, die die folgenden Komponenten enthalten:
a) 0,5 bis 95 Gew.-%, bevorzugt 10 bis 60 Gew.-% und besonders bevorzugt 15 bis 50 Gew.-% (Meth)acrylamidphosphat gemäß Formel (I),
b) 0,01 bis 15 Gew.-%, besonders bevorzugt 0,1 bis 8,0 Gew.-% an Initiator für die radikalische Polymerisation,
c) 0 bis 80 Gew.-%, bevorzugt 0 bis 60 Gew.-% und besonders bevorzugt 10 bis 50 Gew.-% nicht acides Monomer,
d) 0 bis 95 Gew.-%, bevorzugt 0 bis 80 Gew.-% und besonders bevorzugt 20 bis 60 Gew.-% Lösungsmittel,
e) 0 bis 20 Gew.-% Füllstoff.

Die Füllstoffmenge richtet sich besonders nach der gewünschten Anwendung des Dentalwerkstoffs. Für die Anwendung als Adhäsiv sind 0 bis 20 Gew.-%, für die Anwendung als Zement 20 bis 75 Gew.-% Füllstoff bevorzugt.

Andere Additive und Hilfsstoffe werden gegebenenfalls in einer Menge von 0,01 bis 10 Gew.% eingesetzt.

Wenn nicht anders angegeben beziehen sich alle Prozentangaben auf die Gesamtmasse des Werkstoffs.

Die Erfindung wird im Folgenden anhand von Beispielen näher erläutert.

### Ausführungsbeispiele:

### Beispiel 1: Phosphorsäuremono-(1-acryloyl-piperidin-4-yl)-ester (APP)

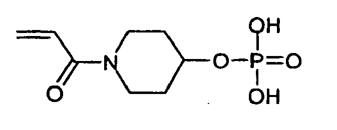

### 1. Stufe: 1-(4-Hydroxy-piperidin-1-yl)-propenon

10.11 g (0.10 mol) 4-Hydroxypiperidin wurden in 60 ml 2M NaOH gelöst. Die Lösung wurde 10 min bei Raumtemperatur gerührt und anschließend auf 0 °C abgekühlt. Eine Lösung von 9.96 g (0.11 mol) Acrylsäurechlorid und 20 mg 2,6-Di-tert.-butyl-4-methylphenol (BHT) in 60 ml Chloroform wurde innerhalb von 2 h bei 0 °C zugetropft. Nach beendeter Zugabe wurde das klare, gelbe Reaktionsgemisch 1 h bei 0 °C gerührt, dann wurde das Eisbad entfernt und die Mischung 3 h bei Raumtemperatur weitergerührt. Organische und wäßrige Phase wurden getrennt. Die wäßrige Phase wurde mit NaCl gesättigt und mit 5x 100 ml Chloroform extrahiert. Die vereinigten organischen Phasen wurden über Na₂SO₄ getrocknet, filtriert, nach Zugabe von 20 mg BHT am Rotationsverdampfer eingeengt und am Feinvakuum getrocknet. Das gelbliche Öl wurde in 5 ml Ethylacetat aufgenommen und mittels Säulenchromatographie gereinigt (Kieselgel 60, 0.035-0.070 mm, Ethylacetat). Das Lösungsmittel wurde nach Zugabe von 25 mg BHT am Rotationsverdampfer eingeengt und am Feinvakuum getrocknet. Man erhielt 4.35 g (28.0 mmol, 28%) eines gelblichen Öls, welches bei der Lagerung im Kühlschrank zu einem gelblichen Feststoff erstarrte.
¹H NMR (CDCl₃, 400 MHz) : δ = 1.47 - 1.63 (m; 2H; CH₂), 1-82 - 1.96 (m; 2H; CH₂), 3.22 - 3.88 (m; 2H; CH₂), 3.77 - 3.88 (m; 1; CH), 3.88 - 3.98 (m; 2H; CH₂), 3.98 - 4.16 (m; 1; OH), 5.68 - 5.70 (dd, *J* = 1.7 Hz, 10.8 Hz; 1H; =CH), 6.20 - 6.24 (dd, *J* = 2.0 Hz, 16.6 Hz; 1H; =CH), 6.56 - 6.63 (dd, *J* = 10.6 Hz, 16.8 Hz; 1H; =CH).
¹³C NMR (CDCl₃, 100 MHz) : δ = 33.7 (CH₂), 34.5 (CH₂), 39.5 (CH₂), 43.2 (CH₂), 66.5 (CH), 127.7 (CH₂), 127.8 (CH), 165.5 (C).

### 2. Stufe: Phosphorsäure(1-acryloyl-piperidin-4-yl)ester-dimethylester

2.60 g (18.0 mmol) Dimethylchlorphosphat, 2.33 g (15.0 mmol) 1-(4-Hydroxy-piperidin-1-yl)-propenon, 10 mg Hydrochinonmonomethylether (MEHQ) und 10 mg BHT wurden unter Argon in 50 ml Dichlormethan gelöst und im Eisbad auf -5 °C abgekühlt. 2.36 g (21.0 mmol) Kalium-tert.-butylat wurden unter Rühren zugegeben. Es wurde 30 min unter Eiskühlung gerührt, wobei sich die Lösung bräunlich verfärbte, dann wurde das Eisbad entfernt und die Mischung für 4 h bei Raumtemperatur gerührt. Anschließend wurden 50 ml gesättigte wäßrige NH₄Cl-Lösung zugegeben. Das Gemisch wurde 10 min gerührt, dann wurden die Phasen getrennt. Die wäßrige Phase wurde mit 2 x 50 ml Dichlormethan extrahiert. Die vereinigten organischen Phasen wurden über Na₂SO₄ getrocknet, filtriert, nach Zugabe von 20 mg BHT am Rotationsverdampfer eingeengt und am Feinvakuum getrocknet. Das braune Öl wurde in 5 ml Dichlormethan aufgenommen und mittels Säulenchromatographie gereinigt (Kieselgel 60, 0.035-0.070 mm, Methylenchlorid/Ethanol 98:2). Der Eluent wurde nach Zugabe von 20 mg BHT am Rotationsverdampfer eingeengt und am Feinvakuum getrocknet. Man erhielt 3.02 g (11.5 mmol; 76%) eines rot-braunen Öls.

| | | | | | | | |
|---|---|---|---|---|---|---|---|
| C₁₀H₁₈NO₅P | Calc. | C | 45.63 | H | 6.89 | N | 5.32 |
| (263.23) | Found | | 43.12 | | 7.19 | | 4.96 |

IR: 3475 (w), 2956 (w, C-H), 1642 (s, C=O), 1608 (s, C=C), 1441 (s, C-H), 1366 (w), 1258 (s, P=O), 1219 (m), 1188 (m), 1000 (vs), 893 (m), 847 (s), 791 (m), 766 (m).
¹H NMR (CDCl₃, 400 MHz) : δ = 1.73 - 1.87 (m; 2H; CH₂), 1.92 - 2.03 (m; 2H; CH₂), 3.40 - 3.64 (m; 2H; CH₂), 3.78 (d, *J* = 11.1 Hz; 6H; O-CH₃), 3. 81 - 3.94 (m; 2H; CH₂), 4.59 - 4.70 (m; 1H; CH), 5.59 (dd, *J* = 2.0 Hz, 10.6 Hz; 1H; =CH), 6.25 (dd, *J* = 2.0 Hz, 16.6 Hz; 1H; =CH), 6.61 (dd, *J* = 10.6 Hz, 16.9 Hz; 1H; =CH).
¹³C NMR (CDCl₃, 100 MHz) : δ = 31.8, 32.8 (CH₂), 38.6 (CH₂), 42.4 (CH₂), 54.3, 54.3 (CH₃), 73.7, 73.7 (CH), 127.6 (CH), 127.8 (CH₂), 165. 4 (C).
³¹P NMR (CDCl₃, 162 MHz) : δ = 1.4.

### 3. Stufe: Phosphorsäuremono-(1-acryloyl-piperidin-4-yl)-ester

1.32 g (5.0 mmol) Phosphorsäuredimethyl-[4-(*N-*acryloyl)piperidyl]ester) und 10 mg BHT wurden unter Argon in 5 ml Dichlormethan gelöst. 1.68 g (11.0 mmol) Bromtrimethylsilan wurden tropfenweise zugegeben. Die klare gelbliche Lösung wurde nach beendeter Zugabe 4 h bei Raumtemperatur gerührt. Zur Entfernung flüchtiger Komponenten wurde die Lösung 45 min bei 45 °C evakuiert. Der ölige gelbe Rückstand wurde mit 5 ml Methanol versetzt. Die klare gelbliche Lösung wurde 18 h bei Raumtemperatur gerührt und anschließend am Rotationsverdampfer eingeengt und am Feinvakuum getrocknet. Das hochviskose gelbliche Öl wurde mit 10 ml Dichlormethan versetzt und bei Raumtemperatur gerührt. Es bildete sich ein leicht gelblicher Feststoff. Dieser wurde abfiltriert und im Vakuumtrockenschrank getrocknet. Der Vorgang wurde mit jeweils 10 ml Ethylacetat und Aceton wiederholt. Der weiße Feststoff wurde mit 5 ml *i*-Propanol versetzt und 18 h bei Raumtemperatur gerührt. Die Suspension wurde filtriert, und das Filtrat wurde am Rotationsverdampfer eingeengt und am Feinvakuum getrocknet. Man erhielt 0.31 g (1.3 mmol; 26%) eines weißen Feststoffs.
¹H NMR (DMSO-d₆, 400 MHz): δ = 1.46 - 1.64 (m; 2H; CH₂), 1.77 - 1.95 (m; 2H; CH₂), 3.24 - 3.47 (m; 2H; N-CH₂), 3.69 - 3.87 (m; 2H; N-CH₂), 4.31 - 4.43 (m; 1H; O-CH), 5.66 (dd, *J* = 2.5 Hz, 10.5 Hz; 1H; =CH), 6.09 (dd, *J* = 2.5 Hz, 16.7 Hz; 1H; =CH), 6.80 (dd, *J* = 10.6 Hz, 16.7 Hz; 1H; =CH).
³¹P NMR (DMSO-d₆, 162 MHz) : δ = -0.6.

### Beispiel 2: 1,3-Bis-(N-acryloyl-N-propyl-amino)-propan-2-yl-dihydrogenphosphat (BAPAPP)

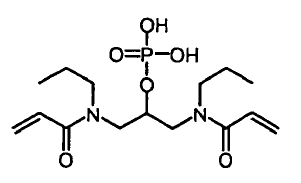

### 1. Stufe: 1,3-Bis-(N-acryloyl-N-propyl-amino)-2-hydroxypropan

17.43 g (0.100 mol) 1,3-Bis-(propylamino)-propan-2-ol wurden mit 110 ml (0.220 mol) 2N Natronlauge versetzt und 10 min bei Raumtemperatur gerührt. Das Gemisch wurde auf 0 °C abgekühlt. Eine Lösung von 19.46 g (0.215 mol) Acrylsäurechlorid und 20 mg BHT in 120 ml Chloroform wurde bei 0 °C innerhalb von 3 h 30 min zugetropft. Nach beendeter Zugabe wurde das Reaktionsgemisch 2 h 30 min bei 0 °C gerührt. Organische und wäßrige Phase wurden getrennt. Die wäßrige Phase wurde mit NaCl gesättigt und mit 5 x 80 ml Methylenchlorid extrahiert. Die vereinigten organischen Phasen wurden über Na₂SO₄ getrocknet, filtriert, nach Zugabe von 20 mg BHT am Rotationsverdampfer eingeengt und am Feinvakuum getrocknet. Das Rohprodukt wurde mittels zweimaliger Säulenchromatographie gereinigt (Kieselgel 60, 0.035-0.070 mm, Ethylacetat). Man erhielt 11.92 g (42.2 mmol; 42%) des reinen Produkts als gelbliches Öl.

| | | | | | | | |
|---|---|---|---|---|---|---|---|
| C₁₅H₂₆N₂O₃ | Calc. | C | 63.80 | H | 9.28 | N | 9.92 |
| (282.39) | Found | | 63.54 | | 9.16 | | 9.67 |

IR: 3349 (m, -OH), 2963 (m), 2933 (m), 2875 (m, C-H), 1641 (vs, C=O), 1603 (vs, C=C), 1427 (vs, C-H), 1370 (m, -O-H), 1271 (w), 1229 (s), 1132 (m, -C-OH), 1059 (m), 977 (s), 957 (m), 895 (w), 794 (s), 745 (m).
¹H NMR (CDCl₃, 400 MHz): δ = 0.86 - 0.93 (m; 6H; CH₃), 1.57 - 1.68 (m; 4H; CH₂-CH₃), 3.25 - 3.53 (m; 8H; CH₂-N), 4.06 - 4.09 (m; 1H; CH), 4.96 - 4.99 (m; 1H; OH), 5.62 - 5.74 (m; 2H; =CH), 6.25 - 6.38 (m; 2H; =CH), 6.54 - 7.00 (m; 2H; =CH).
¹³C NMR (CDCl₃, 100 MHz) : δ = 11.1 (CH₃), 11.4 (CH₃), 20.6 (CH₂), 22.5 (CH₂), 22.6 (CH₂), 48.8 (CH₂), 51.4 (CH₂), 51.6 (CH₂), 51.9 (CH₂), 52.0 (CH₂), 70.0 (CH), 71.7 (CH), 127.2 (CH), 127.3 (CH), 127.6 (CH₂), 128. 4 (CH), 128. 5 (CH₂), 128.7 (CH₂), 167.8 (C).

### 2. Stufe: 1,3-Bis- (N-acryloyl-N-propyl-amino) -propan-2-yl-dimethylphosphat

2.60 g (18.0 mmol) Dimethylchlorphosphat, 4.24 g (15.0 mmol) 1,3-Bis-(N-acryloyl-N-propyl-amino)-2-hydroxypropan, 20 mg MEHQ und 20 mg BHT wurden unter Argon in 80 ml Dichlormethan gelöst und im Eisbad auf -5 °C abgekühlt. 2.36 g (21.0 mmol) Kalium-tert.-butylat wurden unter Rühren zugegeben. Es wurde 30 min unter Eiskühlung gerührt, wobei sich die Lösung leicht gelblich verfärbte, dann wurde das Eisbad entfernt, und die Mischung wurde 4 h bei Raumtemperatur gerührt. Anschließend wurden 80 ml gesättigte wäßrige NH₄Cl-Lösung zugegeben. Das Gemisch wurde 10 min gerührt, dann wurden die Phasen getrennt. Die wäßrige Phase wurde mit 2 x 80 ml Dichlormethan extrahiert. Die vereinigten organischen Phasen wurden über Na₂SO₄ getrocknet, filtriert, nach Zugabe von 30 mg BHT am Rotationsverdampfer eingeengt und am Feinvakuum getrocknet. Das Rohprodukt wurde mittels Säulenchromatographie gereinigt (Kieselgel 60, 0.035-0.070 mm, Ethylacetat/Aceton 90:10 → 80:20). Man erhielt 2.74 g (11.6 mmol; 50%) eines gelblichen Öls.

### 3. Stufe: 1,3-Bis-(N-acryloyl-N-propyl-amino)-propan-2-yl-dihydrogenphosphat

1.17 g (3.0 mmol) 1,3-Bis-(N-acryloyl-N-propyl-amino)-propan-2-yl-dimethylphosphat und 10 mg BHT wurden unter Argon in 5 ml Dichlormethan gelöst. 1.01 g (6.6 mmol) Bromtrimethylsilan wurden tropfenweise zugegeben. Die klare gelbliche Lösung wurde nach beendeter Zugabe 3 h bei Raumtemperatur gerührt. Zur Entfernung flüchtiger Komponenten wurde die Lösung 45 min bei 45 °C evakuiert. Der ölige gelbe Rückstand wurde mit 5 ml Methanol versetzt, und die klare gelbliche Lösung wurde 18 h bei Raumtemperatur gerührt. Die Lösung wurde am Rotationsverdampfer eingeengt und am Feinvakuum getrocknet. Man erhielt 1.21 g (3.3 mmol; 111%) eines leicht gelblichen Schaums.

### Beispiel 3: Untersuchung der Hydrolysestabilität des Phosphorsäuremono-(1-acryloyl-piperidin-4-yl)-ester

Es wurde eine 20%ige Lösung des Phosphorsäuremono-(1-acryloyl-piperidin-4-yl)-esters aus Beispiel 1 stabilisiert mit 200 ppm 2,6-Di-t-butyl-4-methylphenol in D₂O/EtOH-d₆ (1:1) hergestellt, diese bei 37 °C gelagert und ¹H-NMR-spektroskopisch untersucht. Nach einer Standzeit von 3 Monaten konnten keine Veränderungen des ¹H-NMR-Spektrums festgestellt werden.

### Beispiel 4: Herstellung eines lichthärtenden Adhäsives auf der Basis von (Meth)acrylamidphosphaten (MAP)

Zur Untersuchung der Dentinhaftung auf Rinderzahndentin wurden Adhäsive mit folgender Zusammensetzung (Angabe in Gew.-%) hergestellt:

| **MAP [Gew.-%]** | **Comonomere [Gew.-%]** | **H₂O [Gew.-%]** | **Initiator [Gew.-%]** | **Haftwerte [MPa]** |
|---|---|---|---|---|
| 14, 6 **BAPAPP**¹⁾ | **A**²⁾: 9,7; **B**³⁾: 2,1; **C**⁴⁾: 47,6 | 25,0 % | 1,0 % | 10,7 ± 2,6 |
| 14,3 **APP**⁵⁾ | **C:** 45,1; **D**⁶⁾: 9,1; **E**⁷⁾: 5, 3; **F**⁸⁾: 12, 4 | 12,8 % | 1,0% | 11,9 ± 5,4 |

| | | | | |
|---|---|---|---|---|
| ¹⁾ **BAPAPP** = Monomer aus Beispiel 2 | | | | |
| ²⁾ **A** = [1-(Methacryloylamino)propyl]-phosphonsäure | | | | |
| ³⁾ **B** = 6-(N-Acryloylamino)hexan-1-ol | | | | |
| ⁴⁾ **C** = N, N'-Diethyl-1,3-propylen-bisacrylamid | | | | |
| ⁵⁾ **APP** = Monomer aus Beispiel 1 | | | | |
| ⁶⁾ **D** = D,L-2-(Acryloylamino)-bernsteinsäure | | | | |
| ⁷⁾ **E** = N-(5-Hydroxy-pentyl)-methacrylamid | | | | |
| ⁸⁾ **F** = Aerosil-H₂O-Gemisch | | | | |

Rinderzähne wurden so in Kunststoffzylinder eingebettet, daß sich das Dentin und der Kunststoff in einer Ebene befanden. Nach Anschleifen der Prüfkörper wurde mit einem Microbrush eine Schicht Adhäsiv obiger Formulierung 30 s auf die Dentinoberfläche einmassiert, mit einem Luftbläser leicht verblasen und mit einer Photopolymerisationslampe Astralis 7 (Ivoclar Vivadent AG) für 20 s belichtet. Dann wurde auf die Adhäsivschicht ein kommerziell erhältliches, dentales Füllungskomposit (Tetric® Ceram, Icoclar Vivadent AG) aufgebracht und 40 s mit der Lampe Astralis 7 ausgehärtet. Anschließend wurden die Prüfkörper 24 h bei 37 °C in Wasser gelagert und die Scherhaftfestigkeit gemäß der ISO-Richtlinie "ISO 1994-ISO TR 11405: Dental Materials Guidance on Testing of Adhesion to Tooth Structure" bestimmt.

## Patentansprüche

1. Polymerisierbarer Dentalwerkstoff, **dadurch gekennzeichnet, daß** er mindestens ein (Meth)acrylamidphosphat der folgenden allgemeinen Formel (I) enthält: 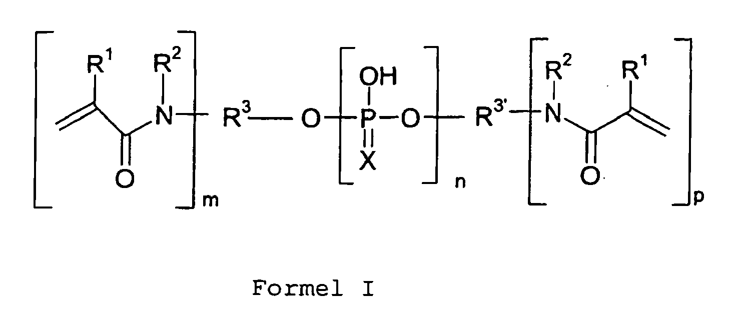 in der
**R¹** H oder CH₃ ist;
**R²** H oder ein C₁-C₉-Alkylrest ist oder zusammen mit dem Stickstoffatom, an das es gebunden ist, und einem oder mehreren Atomen, die zu R³ bzw. R^{3'} gehören, einen heterocyclischen Ring bildet;
**R³, R^{3'}** unabhängig voneinander ein m+n-wertiger bzw. p+n-wertiger, linearer oder verzweigter aliphatischer C₁-C₅₀-Rest, ein m+n-wertiger bzw. p+n-wertiger aromatischer C₆-C₁₈-Rest oder ein m+n-wertiger bzw. p+n-wertiger cycloaliphatischer, araliphatischer oder heterocyclischer C₃-C₁₈-Rest sind, wobei die Kohlenstoffketten des oder der Reste durch O, S, CONR⁴, OCONH unterbrochen sein können, oder zusammen mit einem oder mehreren Atomen, die zu R² gehören und dem Stickstoffatom, an das R² gebunden ist, einen heterocyclischen Ring bilden, wobei R^{3'} H ist, wenn p = 0, und wobei
**R⁴** H, C₁-C₁₀-Alkyl, C₆-C₁₂-Aryl, C₆-C₁₀-Aralkyl oder ein bicyclischer C₄-C₁₂-Rest ist;
**n** 1, 2, 3 oder 4 ist, wenn p = 0, und 1 oder 2 ist, wenn p ≠ 0;
**m** 1, 2, 3 oder 4 ist;
**p** 0, 1, 2, 3 oder 4 ist;
**X** O oder S ist.

2. Dentalwerkstoff nach Anspruch 1, bei dem R² von H verschieden ist.

3. Dentalwerkstoff nach einem der vorhergehenden Ansprüche, bei dem mindestens eine der Variablen ein der folgenden Bedeutungen hat:
**R¹** = H und/oder CH₃, wobei im Falle m > 1 die R¹-Reste unterschiedliche Bedeutungen haben können;
**R²** = H oder ein C₁-C₂₀-Alkylrest oder R² bildet zusammen mit dem Stickstoffatom, an das es gebunden ist, und einem oder mehreren Atomen, die zu R³ bzw. R^{3'} gehören, einen heterocyclischen Ring, vorzugsweise einen Ring mit einem Stickstoffatom und 4 bis 7 Kohlenstoffatomen, insbesondere einen Piperidinylring;
**R³, R^{3'} =** unabhängig voneinander ein m+n-wertiger bzw. n+p-wertiger linearer oder verzweigter aliphatischer C₁-C₁₀-Rest, ein m+n-wertiger bzw. n+p-wertiger aromatischer C₆-C₁₀-Rest oder ein m+n-wertiger bzw. n+p-wertiger C₅-C₈ cycloaliphatischer oder araliphatischer C₆-C₁₂-Rest, wobei die Kohlenstoffketten des oder der Reste durch O unterbrochen sein können, oder R³ und/oder R^{3'} bilden zusammen mit einem oder mehreren Atomen, die zu R² gehören, und dem Stickstoffatom, an das R² gebunden ist, einen heterocyclischen Ring, vorzugsweise einen Ring mit einem Stickstoffatom und 4 bis 7 Kohlenstoffatomen, insbesondere einen Piperidinylring, wobei R^{3'} H ist, wenn p = 0;
**R⁴** = H, C₁-C₆-Alkyl, C₆-Aryl;
**n** = 1 oder 2, wenn p = 0, und 1, wenn p ≠ 0;
**m** = 1 oder 2;
**p** = 0, 1 oder 2;
**X** = O.

4. Dentalwerkstoff nach einem der vorhergehenden Ansprüche, bei dem mindestens eine der Variablen ein der folgenden Bedeutungen hat:
**R¹** = H oder CH₃;
**R²** = H oder ein C₁-C₃-Alkylrest oder R² bildet zusammen mit dem Stickstoffatom, an das es gebunden ist, und einem oder mehreren Atomen, die zu R³ gehören, einen Piperidinylring;
**R³** = ein m+n-wertiger linearer oder verzweigter aliphatischer C₁-C₃-Rest oder R³ bildet zusammen mit einem oder mehreren Atomen, die zu R² gehören, und dem Stickstoffatom, an das R² gebunden ist, einen Piperidinylring, ganz besonders bevorzugt ein C₃-Alkylenrest;
**R³'** = H ;
**R⁴** = H, C₁-C₃-Alkyl;
**n** = 1;
**m** = 1 oder 2;
**p** = 0;
**X** = O.

5. Dentalwerkstoff nach einem der vorhergehenden Ansprüche, der zusätzlich ein Lösungsmittel enthält.

6. Dentalwerkstoff nach Anspruch 5, der als Lösungsmittel Wasser oder eine Mischung von Wasser und einem mit Wasser mischbaren Lösungsmittel enthält.

7. Dentalwerkstoff nach einem der vorhergehenden Ansprüche, der zusätzlich mindestens ein nicht acides radikalisch polymerisierbares Monomer enthält.

8. Dentalwerkstoff nach Anspruch 7, der als nicht acides Monomer ein oder mehrere Monomere aus der folgenden Gruppe enthält: Mono(meth)acrylverbindungen, Mesityl(meth)acrylat, 2-(Alkoyxymethyl)acrylsäuren, 2-(Ethoxymethyl)acrylsäure, 2-(Hydroxymethyl)acrylsäure, N-mono-und N-disubstitiuierte Acrylamide, N-Ethylacrylamid, N,N-Dimethylacrylamid, N-(2-Hydroxyethyl)acrylamid, N-Methyl-N-(2-hydroxyethyl)acrylamid, N-monosubstituierte Methacrylamide, N-Ethylmethacrylamid, N-(2-Hydroxyethyl)methacrylamid, N-Vinylpyrrolidon und Allylether.

9. Dentalwerkstoff nach Anspruch 7 oder 8, der als nicht acides Monomer ein oder mehrere Monomere aus der folgenden Gruppe enthält: Urethane aus 2-(Hydroxymethyl)acrylsäure und Diisocyanten, 2,2,4-Trimethylhexamethylendiisocyanat, Isophorondiisocyanat, vernetzende Pyrrolidone, 1,6-Bis(3-vinyl-2-pyrrolidonyl)-hexan, Bisacrylamide, Methylen- und Ethylenbisacrylamid, Bis(meth)acrylamide, N,N'-Diethyl-1,3-bis(acrylamido)-propan, 1,3-Bis(methacrylamido)-propan, 1,4-Bis(acrylamido)-butan und 1,4-Bis(acryloyl)-piperazin.

10. Dentalwerkstoff nach einem der vorhergehenden Ansprüche, der zusätzlich mindestens einen Initiator für die radikalische Polymerisation enthält.

11. Dentalwerkstoff nach einem der vorhergehenden Ansprüche, der zusätzlich mindestens einen Füllstoff enthält.

12. Dentalwerkstoff nach einem der vorhergehenden Ansprüche, der folgende Komponenten enthält:
a) 0,5 bis 95 Gew.-% (Meth)acrylamidphosphat gemäß Formel (I),
b) 0,01 bis 15 Gew.-% an Initiator,
c) 0 bis 80 Gew.-% nicht acides radikalisch polymerisierbares Monomer,
d) 0 bis 95 Gew.-% Lösungsmittel,
e) 0 bis 20 Gew.-% Füllstoff.

13. Dentalwerkstoff nach Anspruch 12, der zusätzlich
f) 0,01 bis 10 Gew.-% an einem oder mehreren weiteren Additiven enthält.

14. Verwendung eines Dentalwerkstoffs gemäß einem der vorhergehenden Ansprüche als Dentaladhäsiv oder Zement.

15. Verwendung nach Anspruch 14 als Schmelz-Dentin-Adhäsiv.

## Claims

1. Polymerisable dental material, **characterised in that** it comprises at least one (meth)acrylamide phosphate of the following general formula (I): 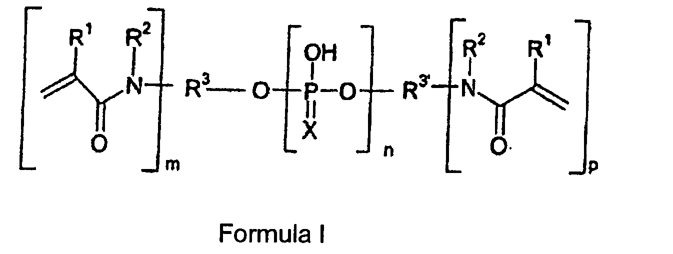 in which
R¹ is H or CH₃;
R² is H or a C₁-C₄ alkyl group or forms a heterocyclic ring together with the nitrogen atom to which it is bonded and one or more atoms which belong to R³ or R^{3'};
R³, R^{3'} independently of each other are a linear or branched aliphatic C₁-C₅₀ group with a valency of m+n or p+n, an aromatic C₆-C₁₈ group with a valency of m+n or p+n, or a cycloaliphatic, araliphatic or heterocyclic C₃-C₁₈ group with a valency of m+n or p+n, wherein the carbon chains of the group or groups can be interrupted by O, S, CONR⁴, OCONH, or form together with one or more atoms which belong to R² and the nitrogen atom to which R² is bonded a heterocyclic ring, wherein R^{3'} is H if p = 0, and wherein
R⁴ is H, C₁-C₁₀ alkyl, C₆-C₁₂ aryl, C₆-C₁₀ aralkyl or a bicyclic C₄-C₁₂ group;
n is 1, 2, 3 or 4 if p = 0, and
is 1 or 2 if p≠0;
m is 1, 2, 3 or 4;
p is 0, 1, 2, 3 or 4;
X is O or S.

2. Dental material according to claim 1, in which R² is different from H.

3. Dental material according to one of the preceding claims, wherein at least one of the variables has one of the following meanings:
R¹ = H and/or CH₃, wherein in the case m > 1 the R¹ groups can have different meanings;
R² = H or a C₁-C₂₀ alkyl group or R² forms together with the nitrogen atom to which it is bonded and one or more atoms which belong to
R³ or R^{3'} a heterocyclic ring, preferably a ring with one nitrogen atom and 4 to 7 carbon atoms, in particular a piperidinyl ring;
R³, R^{3'} = independently of each other a linear or branched aliphatic C₁-C₁₀ group with a valency of m+n or n+p, an aromatic C₆-C₁₀ group with a valency of m+n or n+p, or a cycloaliphatic C₅-C₈ or araliphatic C₆-C₁₂ group with a valency of m+n or n+p, wherein the carbon chains of the group or groups can be interrupted by O, or R³ and/or R^{3'} form together with one or more atoms which belong to R² and the nitrogen atom to which R² is bonded a heterocyclic ring, preferably a ring with one nitrogen atom and 4 to 7 carbon atoms, in particular a piperidinyl ring, wherein R^{3'} is H if p = 0;
R⁴ = H, C₁-C₆ alkyl, C₆ aryl;
n = 1 or 2 if p = 0, and
1 if p≠0;
m = 1 or 2;
p = 0, 1 or 2;
X= O

4. Dental material according to one of the preceding claims, wherein at least one of the variables has one of the following meanings:
R¹ = H or CH₃;
R² = H or a C₁-C₃ alkyl group or R² forms together with the nitrogen atom to which it is bonded and one or more atoms which belong to R³ a piperidinyl ring;
R³ = a linear or branched aliphatic C₁-C₃ group with a valency of m+n, or R³ forms together with one or more atoms which belong to
R² and the nitrogen atom to which the R² is bonded a piperidinyl ring, quite particularly preferably a C₃ alkylene group;
R^{3'} = H;
R⁴ = H, C₁-C₃ alkyl;
n = 1;
m = 1 or 2;
p = 0;
X= O.

5. Dental material according to one of the preceding claims, which additionally comprises a solvent

6. Dental material according to claim 5, which comprises water or a mixture of water and a solvent miscible with water as the solvent.

7. Dental material according to one of the preceding claims, which additionally comprises at least one non-acidic, radically polymerisable monomer.

8. Dental material according to claim 7, which comprises one or more monomers from the following group as the non-acidic monomer: mono(meth)acrylic compounds, mesityl (meth)acrylate, 2-(alkoxymethyl)acrylic acids, 2-(ethoxymethyl)acrylic acid, 2-(hydroxymethyl)acrylic acid, N-mono- and N-disubstituted acrylamides, N-ethyl acrylamide, N,N-dimethyl acrylamide, N-(2-hydroxyethyl)acrylamide, N-methyl-N-(2-hydroxyethyl) acrylamide, N-monosubstituted methacrylamides, N-ethyl methacrylamide, N-(2-hydroxyethyl) methacrylamide, N-vinyl pyrrolidone and allyl ethers.

9. Dental material according to claim 7 or 8, which comprises one or more monomers from the following group as the non-acidic monomer: urethanes from 2-(hydroxymethyl)acrylic acid and diisocyanates, 2,2,4-trimethylhexamethylene diisocyanate, isophorone diisocyanate, crosslinking pyrrolidones, 1,6-bis(3-vinyl-2-pyrrolidonyl)-hexane, bisacrylamides, methylene and ethylene bisacrylamide, bis(meth)acrylamides, N,N-diethyl-1,3-bis(acrylamido)-propane, 1,3-bis(methacrylamido)-propane, 1,4-bis(acrylamido)-butane and 1,4-bis(acryloyl)-piperazine.

10. Dental material according to one of the preceding claims, which additionally comprises at least one initiator for the radical polymerisation.

11. Dental material according to one of the preceding claims, which additionally comprises at least one filler.

12. Dental material according to one of the preceding claims, which comprises the following components:
a) 0.5 to 95 wt.% (meth)acrylamide phosphate according to Formula (I),
b) 0.01 to 15 wt.% initiator,
c) 0 to 80 wt.% non-acidic radically polymerisable monomer,
d) 0 to 95 wt.% solvent,
e) 0 to 20 wt.% filler.

13. Dental material according to claim 12, which additionally comprises
f) 0.01 to 10 wt.% of one or more further additives.

14. Use of a dental material according to any one of the preceding claims as dental adhesive or cement.

15. Use according to claim 14 as an enamel-dentine adhesive.

## Revendications

1. Matériau dentaire polymérisable, **caractérisé en ce qu'**il contient au moins un (méth)acrylamide-phosphate de formule générale (I) suivante : 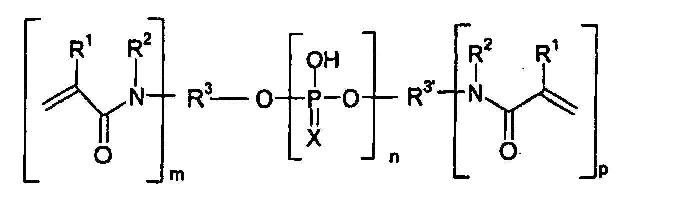 dans laquelle
**R¹** est H ou CH₃ ;
**R²** est H ou un radical alkyle en C₁-C₄ ou forme, conjointement avec l'atome d'azote auquel il est fixé et un ou plusieurs atomes qui appartiennent à R³ ou R^{3'}, un cycle hétérocyclique ;
**R³, R^{3'}** représentent, indépendamment l'un de l'autre, un radical aliphatique en C₁-C₅₀ m+n-valent ou p+n-valent, linéaire ou ramifié, un radical aromatique en C₆-C₁₈ m+n-valent ou p+n-valent ou un radical cyclo-aliphatique, araliphatique ou hétérocyclique en C₃-C₁₈ m+n-valent ou p+n-valent, les chaînes carbonées du radical ou des radicaux pouvant être interrompues par O, S, CONR⁴, OCONH, ou forment, conjointement avec un ou plusieurs atomes qui appartiennent à R² et l'atome d'azote auquel est fixé R², un cycle hétérocyclique, R^{3'} étant H lorsque p = 0, et
**R⁴** est H, un groupe alkyle en C₁-C₁₀, aryle en C₆-C₁₂, aralkyle en C₆-C₁₀ ou un radical bicyclique en C₄-C₁₂ ;
**n** est 1, 2, 3 ou 4, lorsque p = 0, et
est 1 ou 2, lorsque p est ≠ 0 ;
m est 1, 2, 3 ou 4 ;
**p** est 0, 1, 2, 3 ou 4 ;
**x** est O ou S.

2. Matériau dentaire selon la revendication 1, dans lequel R² est différent de H.

3. Matériau dentaire selon l'une quelconque des revendications précédentes, dans lequel au moins l'une des variables a l'une des significations suivantes :
**R¹** est H et/ou CH₃, dans le cas où m > 1, les radicaux R¹ pouvant avoir des significations différentes ;
**R²** est H ou un radical alkyle en C₁-C₂₀ ou R² forme, conjointement avec l'atome d'azote auquel il est fixé et un ou plusieurs atomes qui appartiennent à R³ ou R^{3'}, un cycle hétérocyclique, de préférence un cycle comportant un atome d'azote et de 4 à 7 atomes de carbone, en particulier un cycle pipéridinyle ;
**R³, R^{3'}** représentent, indépendamment l'un de l'autre, un radical aliphatique en C₁-C₁₀ m+n-valent ou n+p-valent, linéaire ou ramifié, un radical aromatique en C₆-C₁₀ m+n-valent ou n+p-valent ou un radical cyclo-aliphatique en C₅-C₈ ou araliphatique en C₆-C₁₂ m+n-valent ou n+p-valent, les chaînes carbonées du radical ou des radicaux pouvant être interrompues par O, ou R³ et/ou R³ forme(nt), conjointement avec un ou plusieurs atomes qui appartiennent à R² et l'atome d'azote auquel est fixé R², un cycle hétérocyclique, de préférence un cycle comportant un atome d'azote et de 4 à 7 atomes de carbone, en particulier un cycle pipéridinyle, R³ étant H lorsque p = 0, et
**R⁴** est H, un groupe alkyle en C₁-C₆, aryle en C₆ ;
**n** est 1 ou 2, lorsque p = 0, et est 1, lorsque p ≠ 0 ;
**m** = 1 ou 2 ;
**p** = 0, 1 ou 2 ;
**x** = O.

4. Matériau dentaire selon l'une quelconque des revendications précédentes, dans lequel au moins l'une des variables a l'une des significations suivantes :
**R¹** est H ou CH₃ ;
**R²** est H ou un radical alkyle en C₁-C₃ ou R² forme, conjointement avec l'atome d'azote auquel il est fixé et un ou plusieurs atomes qui appartiennent à R³, un cycle pipéridinyle ;
**R³** représente un radical aliphatique en C₁-C₃ m+n-valent, linéaire ou ramifié, ou R³ forme, conjointement avec un ou plusieurs atomes qui appartiennent à R² et l'atome d'azote auquel est fixé R², un cycle pipéridinyle, de façon tout particulièrement préférée un radical alkylène en C₃ ;
**R^{3'}** = H ;
**R⁴** = H, un groupe alkyle en C₁-C₃ ;
**n** = 1 ;
**m** = 1 ou 2 ;
**p** = 0 ;
**x** = O.

5. Matériau dentaire selon l'une quelconque des revendications précédentes, qui contient en outre un solvant.

6. Matériau dentaire selon la revendication 5, qui contient en tant que solvant de l'eau ou un mélange d'eau et d'un solvant miscible à l'eau.

7. Matériau dentaire selon l'une quelconque des revendications précédentes, qui contient en outre au moins un monomère n'ayant pas un caractère acide, susceptible de polymérisation radicalaire.

8. Matériau dentaire selon la revendication 7, qui contient en tant que monomère n'ayant pas un caractère acide un ou plusieurs monomères choisis dans le groupe suivant : composés mono(méth)acryliques, (méth)acrylate de mésityle, acides 2-(alcoxyméthyl)acryliques, acide 2-(éthoxyméthyl)acrylique, acide 2-(hydroxyméthyl)acrylique, acrylamides N-mono- et N-disubstitués, N-éthyl-acrylamide, N,N-diméthylacrylamide, N-(2-hydroxyéthyl)acrylamide, N-méthyl-N-(2-hydroxyéthyl)acrylamide, méthacrylamides N-monosubstitués, N-éthyl-méthacrylamide, N-(2-hydroxyéthyl)méthacrylamide, N-vinylpyrrolidone et éther allylique.

9. Matériau dentaire selon la revendication 7 ou 8, qui contient en tant que monomère n'ayant pas un caractère acide un ou plusieurs monomères choisis dans le groupe suivant : uréthannes à base d'acide 2-(hydroxyméthyl)acrylique et de diisocyanates, 2,2,4-triméthylhexaméthylènediisocyanate, isophorone-diisocyanate, pyrrolidones réticulables, 1,6-bis(3-vinyl-2-pyrrolidonyl)-hexane, bis-acrylamides, méthylène- et éthylène-bis-acrylamide, bis(méth)acrylamides, N,N'-diéthyl-1,3-bis(acrylamido)-propane, 1,3-bis(méthacrylamido)-propane, 1,4-bis(acrylamido)-butane et 1,4-bis(acryloyl)-pipérazine.

10. Matériau dentaire selon l'une quelconque des revendications précédentes, qui en outre contient au moins un amorceur pour la polymérisation radicalaire.

11. Matériau dentaire selon l'une quelconque des revendications précédentes, qui en outre contient au moins une charge.

12. Matériau dentaire selon l'une quelconque des revendications précédentes, qui contient les composants suivants :
a) de 0,5 à 95 % en poids de (méth)acrylamide-phosphate selon la formule (I),
b) de 0,01 à 15 % en poids d'amorceur,
c) de 0 à 80 % en poids de monomère susceptible de polymérisation radicalaire,
d) de 0 à 95 % en poids de solvant,
e) de 0 à 20 % en poids de charge.

13. Matériau dentaire selon la revendication 12, qui contient en outre
f) de 0,01 à 10 % en poids d'un ou plusieurs autres additifs.

14. Utilisation d'un matériau dentaire selon l'une quelconque des revendications précédentes, en tant que ciment ou adhésif dentaire.

15. Utilisation selon la revendication 14, en tant qu'adhésif pour émail-dentine.
